# EUROPÄISCHE PATENTSCHRIFT

(11) **EP 1 891 915 B1**
(45) Veröffentlichungstag und Bekanntmachung des Hinweises auf die Patenterteilung: **06.05.2009**
(21) Anmeldenummer: 06405367.1
(22) Anmeldetag: 24.08.2006
(51) Int. Cl.: A61F 9/009

(54) **Ophthalmologische Schutzfolie**
Protective foil for ophthalmic treatment
Dispositif de protection pour traitement ophtalmique

(43) Veröffentlichungstag der Anmeldung: 27.02.2008
(73) Patentinhaber: SIE AG, Surgical Instrument Engineering, 2562 Port (CH)
(72) Erfinder: Kanngiesser, Hartmut, 4500 Solothurn (CH)
(74) Vertreter: Vogel, Dany

(56) Entgegenhaltungen:
- DE-A1- 3 800 076
- US-A1- 2005 143 718

## Beschreibung

### Technisches Gebiet

Die vorliegende Erfindung betrifft eine Schutzfolie zum Schützen einer Augenhornhaut vor einem direkten Kontakt durch einen Laserapplikator.

### Stand der Technik

In der ophthalmologischen Chirurgie wird die Lasertechnologie bereits seit vielen Jahren eingesetzt. Mittels ultrakurzen Laserpulsen, beispielsweise Laserpulse von Picosekundenlasern mit Pulsbreiten von 1 ps bis 10ps (1 ps=10⁻¹²s) oder Laserpulse von Femtosekundenlasern mit Pulsbreiten von 1fs bis 1000fs (1fs=10⁻¹⁵s), kann insbesondere eine Gewebelappen (ein so genanntes Flap) auf der äusseren Oberfläche der Augenhornhaut geschnitten werden, was vorher durch Mikrokeratome mittels mechanischen Klingen vorgenommen wurde. Um das Augengewebe zu schneiden, muss einerseits der Laserstrahl auf einige wenige Mikrometer stark fokussiert werden und müssen andererseits einzelne Laserpulse genau nebeneinander platziert werden. Um eine genügend hohe Präzision zu erreichen, müssen der Laserapplikator und das zu bearbeitende Gebiet des Auges präzise und stabil gekoppelt werden. Allerdings birgt der direkte Augenkontakt mit dem Laserapplikator das Risiko, dass infektiöses Material, beispielsweise Viren, von einem Patienten auf einen anderen übertragen werden, wenn der Laserapplikator nicht genügend desinfiziert wird, oder dass das Augengewebe durch den Laserapplikator mechanisch beschädigt wird.

In der Patentanmeldung US 2005/0143718 wird ein chirurgisches Verfahren zur Behandlung eines Auges mittels ultrakurzen Laserpulsen beschrieben. Um das Auge vor direktem Kontakt mit einem Referenzkörper des Laserapplikators zu schützen, wird eine transparente Folie zwischen das Auge und den Referenzkörper eingeschoben. Gepulste Laserstrahlen werden vom Laserapplikator durch die Schutzfolie hindurch in das Augengewebe projiziert. Die in US 2005/0143718 beschriebene Schutzfolie kann jedoch nicht verhindern, dass bei unsachgemässem Betrieb, beispielsweise bei falscher Programmierung des zu bearbeitenden Gewebegebiets, Augengewebe ausserhalb des tatsächlich zu behandelnden Gebiets ungewollt durch den Laserstrahl zerstört wird.

### Darstellung der Erfindung

Es ist eine Aufgabe der vorliegenden Erfindung eine verbesserte Schutzfolie vorzuschlagen zum Schützen der Augenhornhaut, insbesondere einer menschlichen Augenhornhaut, vor einem direkten Kontakt durch einen Laserapplikator. Dabei soll an dieser Stelle klar festgehalten werden, dass mit dem Begriff Laserapplikator nicht bloss ein Lichtprojektionssystem für gepulste Laserstrahlen, sondern auch ein entfernbar oder fest mit einem Lichtprojektor verbundener Kontaktkörper, gemeint ist, welcher beispielsweise als Applanationskörper, Referenzkörper und/oder Austrittsfenster des Lichtprojektors vorgesehen ist, und durch welchen hindurch die vom Lichtprojektor projizierten Laserstrahlen schlussendlich auf respektive in das Augengewebe projiziert werden.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass die zum Schützen einer Augenhornhaut vor einem direkten Kontakt durch einen Laserapplikator vorgesehene ophthalmologische Schutzfolie einen Bearbeitungsbereich mit einer ersten Foliendicke, beispielsweise mit einem Wert im Bereich von 100µm bis 150µm, und einen bezüglich des Bearbeitungsbereichs erhöhten Profilbereich, welcher dicker als die erste Foliendicke (d₁) ausgestaltet ist, aufweist. Im Zustand, in welchem die Schutzfolie zwischen Augenhornhaut und appliziertem Laserapplikator angeordnet ist, positioniert der Bearbeitungsbereich einen zu behandelnden ersten Teil der Augenhornhaut in einen Fokalbereich des Laserapplikators, und der Profilbereich positioniert einen nicht zu behandelnden zweiten Teil der Augenhornhaut ausserhalb des Fokalbereichs. Das heisst, der gegenüber dem Bearbeitungsbereich der Schutzfolie erhöhte (dickere) Profilbereich drückt den nicht zu behandelnden Augengewebebereich aus der Fokalfläche (Fokalebene, Schnittebene) des Laserapplikators, so dass die fokussierten Laserpulse diesen Bereich nicht erreichen und damit ungewollt beinträchtigen und schädigen können. Die Schutzfolie bildet eine sterile Trennschicht zwischen dem Laserapplikator und dem Auge. Die Schutzfolie erfüllt zudem die Funktion einer Distanzfolie, d.h. sie dient als Distanzhalter zwischen dem Laserapplikator und dem menschlichen Auge. Als Distanzfolie bestimmt die Schutzfolie die Dicke eines von der Augenhornhaut zumindest teilweise abgetrennten Gewebelappens, da die Tiefenpositionierung der Fokalfläche des Laserapplikators im Augengewebe durch die Dicke der Schutzfolie mitbestimmt wird.

In einer bevorzugten Ausführungsvariante ist der Bearbeitungsbereich vom Profilbereich umlaufend begrenzt. Der Bearbeitungsbereich ist beispielsweise zentriert auf der Schutzfolie angeordnet und der Profilbereich ist konzentrisch zum Bearbeitungsbereich angeordnet. Der Bearbeitungsbereich ist beispielsweise kreisrund ausgestaltet ist und weist einen Durchmesser im Bereich von 8mm bis 11 mm auf. Ein vom Profilbereich kreisrund begrenzter Bearbeitungsbereich ermöglicht das Schneiden von Gewebelappen (Flap) in der Augenhornhaut, ohne dass umliegendes Augengewebe von fokussierten Laserpulsen beeinträchtigt werden kann.

In verschiedenen Ausführungsvarianten ist der Profilbereich als Profilrand ausgestaltet, der vom Bearbeitungsbereich abgestuft ist und eine zweite Foliendicke aufweist, oder der Profilbereich ist so ausgestaltet, dass er sich mit einer bezüglich des Bearbeitungsbereichs graduell zunehmenden Foliendicke, also mit einer graduell zunehmenden Erhöhung, an den Bearbeitungsbereich anschliesst. Der Profilrand weist beispielsweise eine zweite Foliendicke mit einem Wert im Bereich von 350µm bis 450µm auf.

Vorzugsweise weist der Profilbereich Druckausgleichöffnungen auf, welche vom Bearbeitungsbereich in einen Innenbereich des Profilbereichs führen. In einer bevorzugten Ausführungsvariante sind die Druckausgleichöffnungen als Rillen im Profilbereich ausgeführt, welche beispielsweise von der Profilwand in einen Innenbereich des Profilrands führen. Beim Anbringen des Laserapplikators auf das Auge werden Fluide (z.B. Luft und Flüssigkeit) zwischen der Schutzfolie und der Augenhornhaut verdrängt. Zudem entstehen beim Schneiden des Augengewebes durch die Laserpulse Gase. Durch die Druckausgleichöffnungen kann ein Überdruck zwischen der Schutzfolie und der Augenhornhaut, insbesondere zwischen dem Profilbereich und der Augenhornhaut, vermieden werden. Die Druckausgleichöffnungen ermöglichen auch das Wegführen von Fluiden, die beim Anbringen des Laserapplikators zwischen der Schutzfolie und der Augenhornhaut verdrängt werden und/oder beim Schneiden entstehen.

Vorzugsweise weist die Seite der Schutzfolie, welche im applizierten Zustand am Laserapplikator anliegt, eine plane Oberfläche auf. Insbesondere wenn die Schutzfolie angefeuchtet wird, ermöglicht die plane Oberfläche eine reibungsarme Bewegung auf dem Laserapplikator.

In einer Ausführungsvariante weist die Schutzfolie einen den Profilbereich umgehenden Spannrand auf, welcher zur Befestigung der Schutzfolie an einem Folienträger vorgesehen ist.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt einen schematischen Querschnitt einer Schutzfolie im applizierten Zustand zwischen der Augenhornhaut und einem Laserapplikator.
Figur 2a zeigt eine Aufsicht der Schutzfolie, welche Druckausgleichöffnungen illustriert, die vom Bearbeitungsbereich in den Innenbereich des Profilbereichs führen.
Figur 2b zeigt einen Querschnitt der Schutzfolie entlang der Querschnittlinie C in der Figur 2a.
Figur 3a zeigt einen schematischen Querschnitt der Schutzfolie mit einem als abgestufter Profilrand ausgestalteten Profilbereich.
Figur 3b zeigt einen schematischen Querschnitt der Schutzfolie mit einem Profilbereich, der sich mit graduell zunehmender Foliendicke an den Bearbeitungsbereich anschliesst.
Figur 3c zeigt einen schematischen Querschnitt der Schutzfolie mit einem weiteren Profilbereich, der sich mit graduell zunehmender Foliendicke an den Bearbeitungsbereich anschliesst.

### Wege zur Ausführung der Erfindung

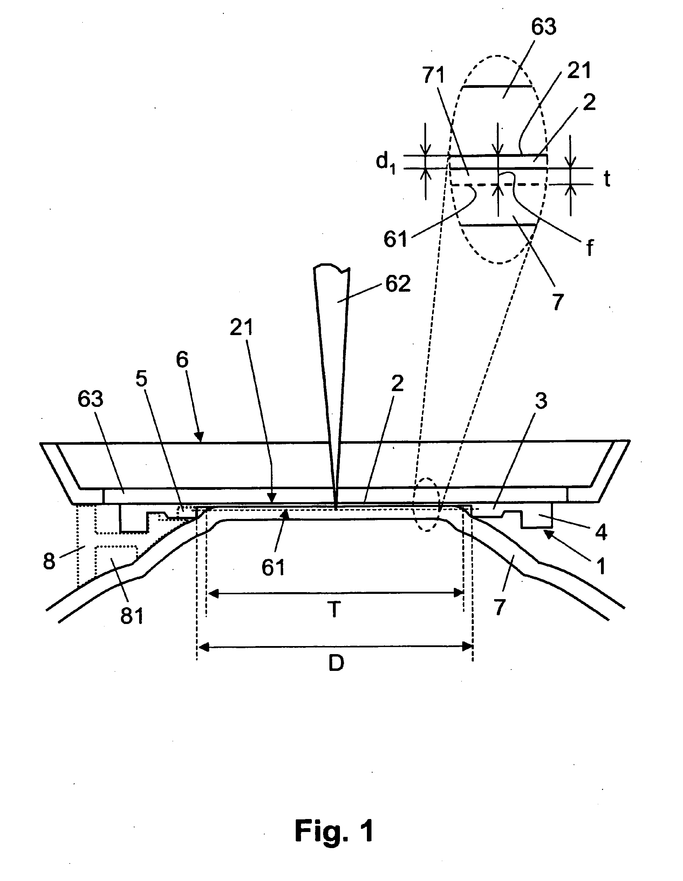

In der Figur 1 bezeichnet, dass Bezugszeichen 6 einen Laserapplikator, welcher eingerichtet ist, gepulste Laserstrahlen 62 auf einen Fokalbereich 61, beispielsweise eine ebene Fokalfläche (Schnittebene), zu projizieren. Der Laserapplikator 6 umfasst einen fest oder entfernbar verbundenen transparenten Kontaktkörper 63, der beispielsweise als Austrittsfenster, Referenzkörper und/oder Applanationskörper vorgesehen ist. Der Kontaktkörper 63 weist beispielsweise eine plane, konkave oder konvexe Kontaktfläche auf.

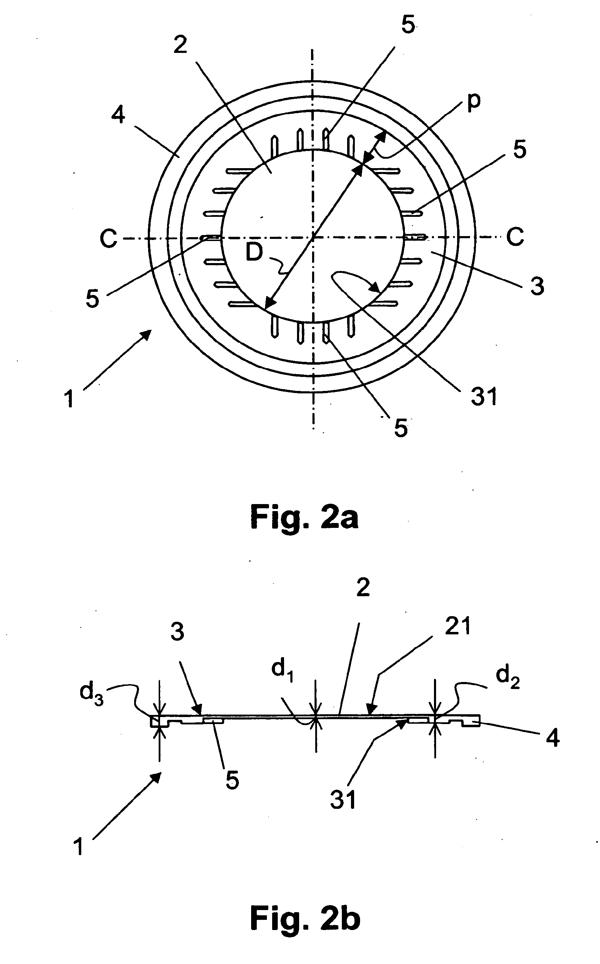

In den Figuren 1, 2a und 2b bezeichnet das Bezugszeichen 1 eine Schutzfolie, welche vorzugsweise im Spritzgussverfahren einstückig aus flexiblem, biokompatiblem Material hergestellt wird, das zumindest für den Wellenlängenbereich um 1000nm hoch transparent ist. Das Material ist beispielsweise ein thermoplastisches Elastomer.

Wie aus der Figur 1 ersichtlich ist, ist die Schutzfolie 1 im applizierten Zustand zwischen den Laserapplikator 6 und das Auge, insbesondere die Augenhornhaut 7, eingefügt. Die Schutzfolie 1 weist eine Seite mit einer planen Oberfläche 21 auf, welche am Laserapplikator 6 respektive an dessen Kontaktkörper 63 anliegt.

Wie in den Figuren 1, 2a, 2b, 3a, 3b und 3c dargestellt ist, umfasst die Schutzfolie 1 einen Bearbeitungsbereich 2, ein den Bearbeitungsbereich 2 begrenzendes Randprofil, welches als Profilbereich 3 bezeichnet wird, und einen Spannrand 4.

Der Bearbeitungsbereich 2 ist vorzugsweise als Film(-bereich) zentriert auf der Schutzfolie 1 angeordnet, ist kreisrund ausgestaltet und weist einen Durchmesser D mit einem Wert im Bereich von 8mm bis 11 mm und eine Dicke d₁ mit einem Wert im Bereich von 100µm bis 150µm auf. Für das Schneiden von Gewebelappen 71 der Augenhornhaut 7 mit unterschiedlicher Lappendicke t und unterschiedlichem Lappendurchmesser T werden vorzugsweise verschiedene Schutzfolien 1 bereitgestellt mit Dicken d₁ von beispielsweise 110µm ±5µm oder 140µm ±5µm und mit Durchmessern D von beispielsweise 8.5mm, 9.0mm, 9.5mm oder 10.0mm. Bei einer die Schnitttiefe bestimmenden Distanz f des Fokalbereichs 61 (Fokalebene) vom Laserapplikator 6, respektive von dessen Kontaktkörper 63, von beispielsweise 250µm ergibt sich bei einem Bearbeitungsbereich 2 mit einer Dicke d₁ von 110µm (140µm) ein Gewebelappen 71 mit einer Lappendicke t von 140µm (110µm).

Der Profilbereich 3 ist vorzugsweise konzentrisch zum Bearbeitungsbereich 2 angeordnet, ringförmig ausgestaltet und weist beispielsweise einen äusseren Durchmesser von 14mm bis 16mm auf.

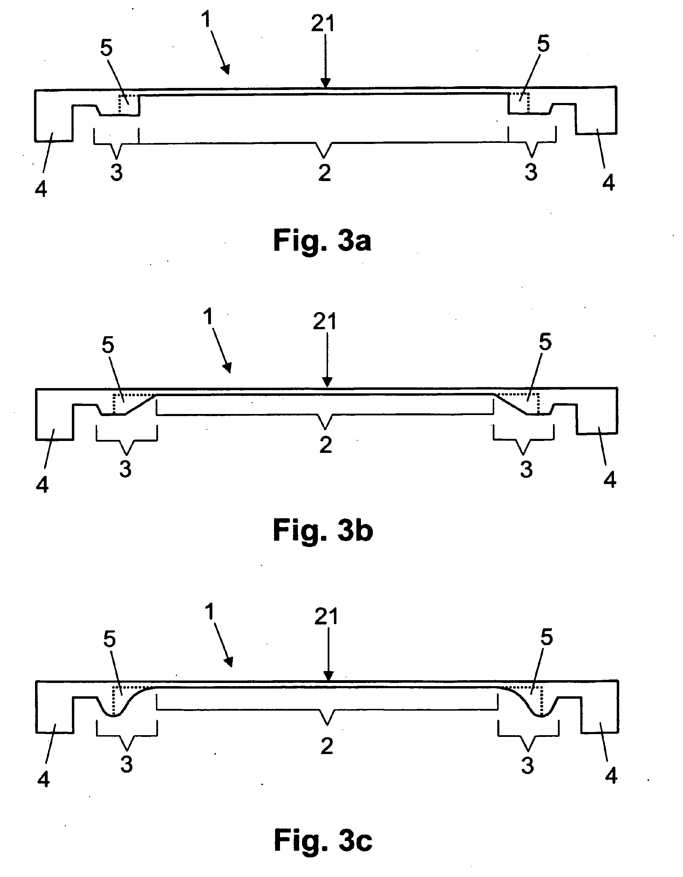

Der Profilbereich 3 ist bezüglich des Bearbeitungsbereichs 2 erhöht. In der Ausführungsvariante gemäss den Figuren 1, 2b und 3a ist der Profilbereich 3 als Profilrand ausgestaltet, der vom Bearbeitungsbereich 2 abgestuft ist und beispielsweise eine Dicke d₂ mit einem Wert im Bereich von 350µm bis 450µm aufweist. Wie in den Figuren 3b und 3c dargestellt ist, ist der Profilbereich 3 in weiteren Ausführungsvarianten so ausgestaltet, dass sein Höhenprofil kontinuierlich an den Bearbeitungsbereich 2 anschliesst und seine Dicke im Anschlussbereich bezüglich der Foliendicke d₁ des Bearbeitungsbereichs 2 graduell zunimmt. Das heisst der Profilbereich 3 schliesst sich mit einer bezüglich des Bearbeitungsbereichs 2 graduell zunehmenden Foliendicke an den Bearbeitungsbereich 2 an. Der Profilbereich 3 kann somit so ausgestaltet sein, dass er mit einer abgestuften Erhöhung (Figur 3a), mit einer geradlinig, linear zunehmenden Erhöhung (Figur 3b), oder mit einer gekrümmt, nicht-linear zunehmenden (konkav, konvex) Erhöhung (Figur 3c) an den Bearbeitungsbereich 2 anschliesst.

Der Profilbereich 3 weist Druckausgleichöffnungen 5 auf, die vom Bearbeitungsbereich 2 in den Innenbereich des Profilbereichs 3 führen, wo sie beispielsweise in eine umlaufende Nut münden. Die Druckausgleichöffnungen 5 sind beispielsweise als Rillen im Profilbereich 3 ausgestaltet, welche beispielsweise im Profilrand von der Profilwand 31 in den Innenbereich des Profilrands 3 führen, eine Tiefe e=d₃-d₁ bis auf das Niveau des Bearbeitungsbereichs 2 aufweisen und bis ungefähr in die Mitte der Breite p des Randprofils 3 führen. In einer alternativen Ausführungsvariante sind die Druckausgleichöffnungen als umlaufende Nut in den Profilbereich 3 eingelassen.

Wie aus der Figur 1 ersichtlich ist, wird die Augenhornhaut 7 im Bereich des Profilbereichs 3 durch das Randprofil vom Laserapplikator 6 respektive von dessen Kontaktkörper 63 weg, aus dem Fokalbereich 61 weggedrückt, so dass die Augenhornhaut 7 in diesem Bereich ausserhalb der Schnittebene des Lasers liegt und sich bei einem Durchmesser D des Bearbeitungsbereichs 2 ein Gewebelappen mit einem Lappendurchmesser T<D ergibt. Der Lappendurchmesser T wird durch die Form und Dimensionierung des Profilbereichs 3 bestimmt. Da der Profilbereich 3 um den Bearbeitungsbereich 2 herum dicker ist als der Abstand f der Fokalebene 61 von der Oberfläche des Laserapplikators 6 respektive dessen Kontaktkörpers 63, schneidet der Laserstrahl 62 somit aus der Augenhornhaut 7 heraus in den Profilbereich 3 der wegwerfbaren Schutzfolie 1.

Der Spannrand 4 schliesst die Schutzfolie 1 ab, ist vorzugsweise konzentrisch zum Profilbereich 3 und Bearbeitungsbereich 2 angeordnet, ringförmig ausgestaltet, und weist beispielsweise einen äusseren Durchmesser von 17mm bis 20mm und eine Dicke d₃ mit einem Wert im Bereich von 450µm bis 550µm auf. Wie in der Figur 1 dargestellt ist, ist die Schutzfolie 1 mittels des Spannrands 4 an einem Folienträger befestigt. Die Schutzfolie 1 ist insbesondere mittels des Spannrands 4 über einen ringförmigen Absatz eines schematisch dargestellten Saugrings 8 gespannt und dadurch entfernbar am Saugring 8 angebracht. Die Druckausgleichöffnungen 5 sind so ausgestaltet und angeordnet, dass sie in entsprechende Öffnungen im Saugring 8 münden. In einer Ausführungsvariante sind die Druckausgleichöffnungen 5 so mit dem Saugring 8 verbunden, dass sie in Fluidkommunikation mit der Unterdruckkammer 81 des Saugrings 8 stehen.

Die Schutzfolie 1 kann auch ohne Spannrand 4 ausgeführt, fest mit einem wegwerfbaren Saugring verbunden sein.

In der vorliegenden Beschreibung wurde bloss eine kreisrunde konzentrische Ausgestaltung und Anordnung des Bearbeitungsbereichs 2 und des Profilbereichs 3 beschreiben, der Fachmann wird jedoch verstehen, dass auch andere Anordnungen und Formgestaltungen möglich sind. Zum Beispiel kann der Bearbeitungsbereich 2 oval oder rechteckig ausgestaltet sein und/oder durch den Profilbereich 3 auf einer oder mehreren Seiten geradlinig begrenzt sein.

## Patentansprüche

1. Schutzfolie (1) zum Schützen einer Augenhomhaut (7) vor einem direkten Kontakt durch einen Laserapplikator (6), **gekennzeichnet durch**
einen Bearbeitungsbereich (2) mit einer ersten Foliendicke (d₁), welcher, zwischen Augenhomhaut (7) und appliziertem Laserapplikator (6) angeordnet, einen ersten Teil der Augenhomhaut (7) in einen Fokalbereich (61) des Laserapplikators (6) positioniert, und
einen bezüglich des Bearbeitungsbereichs (2) erhöhten Profilbereich (3), welcher, zwischen Augenhomhaut (7) und appliziertem Laserapplikator (6) angeordnet, einen zweiten Teil der Augenhomhaut (7) ausserhalb des Fokalbereichs (61) positioniert.

2. Schutzfolie (1) nach Anspruch 1, **dadurch gekennzeichnet, dass** der Bearbeitungsbereich (2) vom Profilbereich (3) umlaufend begrenzt ist.

3. Schutzfolie (1) nach einem der Ansprüche 1 oder 2, **dadurch gekennzeichnet, dass** der Profilbereich (3) als vom Bearbeitungsbereich (2) abgestufter Profilrand mit einer zweiten Foliendicke (d₂) ausgestaltet ist.

4. Schutzfolie (1) nach einem der Ansprüche 1 oder 2, **dadurch gekennzeichnet, dass** der Profilbereich (3) sich mit einer bezüglich des Bearbeitungsbereichs (2) graduell zunehmenden Foliendicke an den Bearbeitungsbereich (2) anschliesst.

5. Schutzfolie (1) nach einem der Ansprüche 1 bis 4, **dadurch gekennzeichnet, dass** der Profilbereich (3) Druckausgleichöffnungen (5) aufweist, welche vom Bearbeitungsbereich (2) in einen Innenbereich des Profilbereichs (3) führen.

6. Schutzfolie (1) nach Anspruch 5, **dadurch gekennzeichnet, dass** die Druckausgleichöffnungen (5) als Rillen im Profilbereich (3) ausgeführt sind.

7. Schutzfolie (1) nach einem der Ansprüche 1 bis 6, **dadurch gekennzeichnet, dass** die Schutzfolie (1) eine Seite mit einer planen Oberfläche (21) aufweist, welche im applizierten Zustand am Laserapplikator (6) anliegt.

8. Schutzfolie (1) nach einem der Ansprüche 1 bis 7, **dadurch gekennzeichnet, dass** die Schutzfolie (1) einen den Profilbereich (3) umgehenden Spannrand (4) aufweist zur Befestigung der Schutzfolie (1) an einem Folienträger.

9. Schutzfolie (1) nach einem der Ansprüche 1 bis 8, **dadurch gekennzeichnet, dass** der Bearbeitungsbereich (2) kreisrund ausgestaltet ist und einen Durchmesser (D) im Bereich von 8mm bis 11 mm aufweist.

10. Schutzfolie (1) nach einem der Ansprüche 1 bis 9, **dadurch gekennzeichnet, dass** der Bearbeitungsbereich (2) auf der Schutzfolie (1) zentriert angeordnet ist, und dass der Profilbereich (3) konzentrisch zum Bearbeitungsbereich (2) angeordnet ist.

## Claims

1. Protective film (1) for protecting a cornea (7) against direct contact with a laser applicator (6), **characterized by** a treatment area (2) of a first film thickness (d1) that, arranged between the cornea (7) and applied laser applicator (6), positions a first part of the cornea (7) in a focal region (61) of the laser applicator (6), and by a profile region (3) that is raised with reference to the treatment area (2) and, arranged between the cornea (7) and applied laser applicator (6), positions a second part of the cornea (7) outside the focal region (61).

2. Protective film (1) according to Claim 1, **characterized in that** the treatment area (2) is limited circumferentially by the profile region (3).

3. Protective film (1) according to either of Claims 1 and 2, **characterized in that** the profile region (3) is configured as a profile rim that is stepped away from the treatment area (2) and has a second film thickness (d2).

4. Protective film (1) according to either of Claims 1 and 2, **characterized in that** the profile region (3) adjoins the treatment area (2) with a film thickness increasing gradually with reference to the treatment area (2).

5. Protective film (1) according to one of Claims 1 to 4, **characterized in that** the profile region (3) has pressure equalizing openings (5) that lead from the treatment area (2) into an inner region of the profile region (3).

6. Protective film (1) according to Claim 5, **characterized in that** the pressure equalizing openings (5) are embodied as grooves in the profile region (3).

7. Protective film (1) according to one of Claims 1 to 6, **characterized in that** the protective film (1) has a side with a flat surface (21) that bears against the laser applicator (6) in the applied state.

8. Protective film (1) according to one of Claims 1 to 7, **characterized in that** the protective film (1) has a clamping rim (4) surrounding the profile region (3) for fastening the protective film (1) on a film carrier.

9. Protective film (1) according to one of Claims 1 to 8, **characterized in that** the treatment area (2) is of circular configuration and has a diameter (D) in the range of 8 mm to 11 mm.

10. Protective film (1) according to one of Claims 1 to 9, **characterized in that** the treatment area (2) is arranged centered on the protective film (1) and **in that** the profile region (3) is arranged concentrically with the treatment area (2).

## Revendications

1. Feuille de protection (1) destinée à protéger la cornée (7) d'un oeil d'un contact direct avec un applicateur laser (6), **caractérisée par**
une zone de traitement (2) qui présente une première épaisseur de feuille (d1) et qui, lorsqu'elle est disposée entre la cornée de l'oeil (7) et l'applicateur laser (6) appliqué, place une première partie de la cornée de l'oeil (7) dans une zone focale (61) de l'applicateur laser (6) et
par une partie profilée (3) surélevée par rapport à la zone de traitement (2) et qui, lorsqu'elle est disposée entre la cornée de l'oeil (7) et l'applicateur laser (6) appliqué, positionne une deuxième partie de la cornée de l'oeil (7) à l'extérieur de la zone focale (61).

2. Feuille de protection (1) selon la revendication 1, **caractérisée en ce que** la périphérie de la zone de traitement (2) est délimitée par la partie profilée (3).

3. Feuille de protection (1) selon l'une des revendications 1 ou 2, **caractérisée en ce que** la partie profilée (3) est configurée comme bord profilé en gradin par rapport à la zone de traitement (2) et dotée d'une deuxième épaisseur de feuille (d2).

4. Feuille de protection (1) selon l'une des revendications 1 ou 2, **caractérisée en ce que** la partie profilée (3) se raccorde à la zone de traitement (2) par une épaisseur de feuille qui augmente progressivement par rapport à celle de la zone de traitement (2).

5. Feuille de protection (1) selon l'une des revendications 1 à 4, **caractérisée en ce que** la partie profilée (3) présente des ouvertures (5) d'équilibrage de pression qui relient la zone de traitement (2) à une partie intérieure de la partie profilée (3).

6. Feuille de protection (1) selon la revendication 5, **caractérisée en ce que** les ouvertures (5) d'équilibrage de pression sont configurées comme rainures ménagées dans la partie profilée (3).

7. Feuille de protection (1) selon l'une des revendications 1 à 6, **caractérisée en ce que** la feuille de protection (1) présente une face à surface (21) plane qui repose sur l'applicateur laser (6) lorsque ce dernier est appliqué.

8. Feuille de protection (1) selon l'une des revendications 1 à 7, **caractérisée en ce que** la feuille de protection (1) présente un bord de serrage (4) qui entoure la partie profilée (3), pour fixer la feuille de protection (1) sur un support de feuille.

9. Feuille de protection (1) selon l'une des revendications 1 à 8, **caractérisée en ce que** la zone de traitement (2) a une configuration circulaire et un diamètre (D) compris dans la plage de 8 mm à 11 mm.

10. Feuille de protection (1) selon l'une des revendications 1 à 9, **caractérisée en ce que** la zone de traitement (2) est disposée au centre de la feuille de protection (1) et **en ce que** la partie profilée (3) est disposée concentriquement par rapport à la zone de traitement (2).
